(19) Europäisches Patentamt
European Patent Office
Office européen des brevets

(11) **EP 3 668 321 B1**

(12) # EUROPEAN PATENT SPECIFICATION

(45) Date of publication and mention
of the grant of the patent:
**29.05.2024 Bulletin 2024/22**

(21) Application number: **18755792.1**

(22) Date of filing: **14.08.2018**

(51) International Patent Classification (IPC):
*A21D 8/04* (2006.01)    *A21D 10/00* (2006.01)
*A21D 13/062* (2017.01)    *C12N 15/81* (2006.01)
*A23L 33/20* (2016.01)

(52) Cooperative Patent Classification (CPC):
**A21D 8/042; A21D 8/047; A21D 10/005;
A21D 13/062; A23L 33/20**

(86) International application number:
**PCT/EP2018/071971**

(87) International publication number:
**WO 2019/034630 (21.02.2019 Gazette 2019/08)**

(54) **WHOLEMEAL BREAD WITH REDUCED FODMAP CONTENT**

VOLLKORNMEHLBROT MIT REDUZIERTEM FODMAP-GEHALT

PAIN COMPLET À TENEUR FODMAP RÉDUIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2017 GB 201713145**

(43) Date of publication of application:
**24.06.2020 Bulletin 2020/26**

(73) Proprietors:
• **Katholieke Universiteit Leuven**
**3000 Leuven (BE)**
• **VIB VZW**
**9052 Gent (BE)**

(72) Inventors:
• **COURTIN, Christophe**
**3012 Wilsele (BE)**
• **STRUYF, Nore**
**2860 Sint-Katelijne-Waver (BE)**
• **THEVELEIN, Johan**
**3052 Oud-Heverlee (Blanden) (BE)**
• **VERSTREPEN, Kevin**
**3001 Leuven (BE)**

(74) Representative: **Arnold & Siedsma**
**Bezuidenhoutseweg 57**
**2594 AC The Hague (NL)**

(56) References cited:
EP-A1- 2 103 219    EP-A1- 3 266 318
WO-A1-2017/220864    FR-A1- 2 988 565

• **MARTINBIANCO F ET AL: "Sensory evaluation of natural fermentation breads with innovative starter cultures", CIENCIA RURAL, UNIVERSIDADE FEDERAL DE SANTA MARIA, BR, vol. 43, no. 9, 1 January 2013 (2013-01-01), pages 1701-1706, XP009508329, ISSN: 0103-8478**
• **DIMITRA DIMITRELLOU ET AL: "Evaluation of thermally-dried Kluyveromyces marxianus as baker's yeast", FOOD CHEMISTRY, ELSEVIER LTD, NL, vol. 115, no. 2, 15 July 2009 (2009-07-15), pages 691-696, XP002690240, ISSN: 0308-8146, DOI: 1016/J.FOODCHEM.2008.12.050 [retrieved on 2008-12-16]**
• **NORE STRUYF ET AL: "Saccharomyces cerevisiae and Kluyveromyces marxianus Cocultures Allow Reduction of Fermentable Oligo-, Di-, and Monosaccharides and Polyols Levels in Whole Wheat Bread", JOURNAL OF AGRICULTURAL AND FOOD CHEMISTRY, vol. 65, no. 39, 22 September 2017 (2017-09-22), pages 8704-8713, XP055510042, US ISSN: 0021-8561, DOI: 10.1021/acs.jafc.7b02793**

- NORE STRUYF ET AL: "Kluyveromyces marxianus yeast enables the production of low FODMAP whole wheat breads", FOOD MICROBIOLOGY., vol. 76, 3 May 2018 (2018-05-03), pages 135-145, XP055510458, GB ISSN: 0740-0020, DOI: 10.1016/j.fm.2018.04.014
- Anonymous: "Functional food opportunity in bread, pizza and baked goods with KluyBread", , 14 December 2017 (2017-12-14), pages 1-15, XP055510267, Retrieved from the Internet: URL:https://turval.com/products/nutrition/bread/180607-kluybread-eng-.pdf [retrieved on 2018-09-26]
- J.G. Muir ET AL: "Reduced FODMAPS in gluten-free grains may explain the improved symptoms in people with IBS following a gluten-free diet", Journal of Nutrition & Intermediary Metabolism, vol. 1, 1 December 2014 (2014-12-01), pages 14-15, XP055510061, ISSN: 2352-3859, DOI: 10.1016/j.jnim.2014.10.043
- STRUYF NORE ET AL: "Kluyveromyces marxianusyeast enables the production of low FODMAP whole wheat breads", FOOD MICROBIOLOGY, ACADEMIC PRESS LTD, LONDON, GB, vol. 76, 3 May 2018 (2018-05-03), pages 135-145, XP085456716, ISSN: 0740-0020, DOI: 10.1016/J.FM.2018.04.014

**Description**

**FIELD OF THE INVENTION**

Disclosed herein is the preparation of wholemeal bread with reduced FODMAP content. The present invention relates to the use of *Kluyveromyces* yeast strains in baking bread.

**BACKGROUND OF THE INVENTION**

The irritable bowel syndrome (IBS) is a chronic functional gastrointestinal disorder with a prevalence of up to 12% in the European population. Typical IBS symptoms are abdominal pain, bloating and altered bowel habits combined with intermittent diarrhoea or constipation. Dietary habits are believed to play a major role in pathogenesis of IBS and a diet low in specific short-chain carbohydrates has been shown to markedly alleviate IBS symptoms in numerous cases. The presumably responsible carbohydrates are known by their acronym 'FODMAPs', meaning fermentable oligo-, di-, and monosaccharides and polyols. FODMAPs represent a group of short-chain carbohydrates and sugar alcohols comprising fructose, lactose, fructo- and galacto-oligosaccharides (fructans and galactans) and polyols. Patients suffering from IBS but also Inflammatory Bowel Disease (IBD) or non-celiac gluten sensitivity (NCGS) are advised to follow a diet that is limited in FODMAPs, which includes that they should avoid the consumption of wheat and wheat-derived products like bread since wheat contains relatively high fructan levels and is therefore a major source of FODMAPs. Fructans are linear or branched polymers consisting mainly or exclusively of fructose units and maximally one glucose unit per molecule. The cut-off value that is considered at-risk of inducing symptoms in people that are sensitive to FODMAPs is > 0.2 g of fructan per serve, and > 0.5 g fructose in excess of glucose per 100 g. These values are, however, not strict values and differ between patients.

Wheat grains contain between 0.7 and 2.9% fructans. Wheat grain fructans have a branched structure and their mean DP ranges from 4 to 6. Wheat wholemeal contains higher fructan levels than wheat flour. During bread dough fermentation wheat grain fructans are enzymatically degraded to glucose and fructose. Despite this degradation, the fructan level in wheat bread is described in literature to be above the cut-off value (0.2 g per serve) that is considered at-risk of inducing symptoms in IBS patients.

Grain based products, such as wheat, oat, barley and rye, are nutritious staple foods and avoiding their consumption may result in nutritional deficiencies. Therefore, adaptations of the bread making process that allow full degradation of fructan in bread and hence elimination/reduction of FODMAPs are desirable. However, prolonged proofing times (>4 h) allowed to effectively diminish FODMAP levels of the final wheat product by levels up to 90% [Ziegler et al. (2016) J. Functional Foods 25, 257-266]. Prolonged proofing times are not desirable from an economical point of view. WO2015/117182 describes a method to produce food products that are low in FODMAP content by using a reconstituted low-FODMAPS wheat gluten protein-based flour (WGPF). The low-FODMAPS WGPF is produced by dispersing wheat flour in an aqueous solution, subsequently recovering wheat gluten proteins from this dispersion, washing the wheat gluten protein resulting in depletion of FODMAPs and combining this with one or more soluble and insoluble materials which have been recovered either from wheat flour or other non-wheat sources and which have been sufficiently depleted of FODMAPS. This low FODMAPs WGPF can be used to produce food products such as bread with a reduced FODMAP content. Although FR2988565 describes the use of *K. marxianus* in white bread in order to adapt the taste characteristics, this publication does not suggest the use of *K. marxianus* in wholemeal bread in order to reduce FODMAP content in wholemeal bread. The use of *K. marxianus* in rye sourdough has been equally described (Plessas et al. (2006) Food Chem. 106, 985-990).

WO2016/113465 describes a method for producing low-fructan grain material by means of lactic acid bacteria.

Laaitikan (2016) Aliment Pharmacol Ther. 44, 460-470, describe a reduction in fructans in rye bread fermented with sourdough.

There is a need for wholemeal bread with reduced FODMAP content, and convenient methods to prepare such bread.

**SUMMARY OF THE INVENTION**

The degradation of grain FODMAPs during wholemeal bread making in a time-efficient manner without alteration of the time needed for the bread making process or with the use of wholemeal flour which leads to final products low in FODMAPs, has never been described.

The present invention relates to a method for reducing fermentable oligo-, di-, and monosaccharides and polyols (FODMAP) levels in grain-based food products by addition of *K. marxianus* to the food making process. Unexpectedly, addition of the yeast *K. marxianus* to the bread making process without alteration of the bread making process enabled significant reduction of FODMAP levels in a wholemeal bread which has inherent high FODMAP levels, while retaining high fibre levels. The FODMAP levels in this bread are below the threshold levels of 0.2 gram per 50 gram bread serving enabling the consumption of this bread by persons suffering from IBS but also Inflammatory Bowel Disease (IBD) or non-celiac gluten sensitivity (NCGS). In a first aspect, the present invention relates to the use of *K. marxianus* in the preparation of wholemeal bread with reduced FODMAP content. In a further aspect, the present invention relates to a wholemeal bread with a FODMAP content of maximum 0.2 gram FODMAP per 50 g of dry bread.

The present invention has the advantage that wholemeal bread can be prepared with a substantially lower FODMAP content than prior art methods, and while retaining high fibre levels.

The present invention has the advantage that wholemeal bread with low FODMAP can be prepared without the use of sourdough or without lengthy proofing periods of up to 4 hours using *Saccharomyces* strains.

The present invention has the advantage that wholemeal bread with low FODMAP can be prepared using wholemeal "as such" and overcomes the cumbersome process of reconstituting a meal.

The present invention has the advantage that individuals with disorders such as IBD and IBS can enjoy a wholemeal bread which has been minimally processed.

Disclosed herein is the use of the yeast *Kluyveromyces marxianus* in the preparation of wholemeal bread with reduced FODMAP (fermentable oligo-, di-, monosaccharides and polyols) content.

In embodiments thereof *K. marxianus* is the strain with deposit number CBS6014. In embodiments of the use *cerevisiae* and *K. marxianus* are used.

In embodiments of the use, *cerevisiae* and *K. marxianus* are used in about equal amounts.

In embodiments of the use the wholemeal comprises maximum 10 g/100 g flour sucrose and/or maximum 10 g/100 g flour glucose and/or maximum 10 g/100 g flour lactose. If both sucrose and glucose are added or if both sucrose and lactose are added or if both glucose and lactose are added or if sucrose and glucose and lactose are added, the total amount of these sugars is at most 10 g/100g flour.

In embodiments of the use the wholemeal comprises maximum 15U/g dm amyloglucosidase.

Disclosed herein is wholemeal bread with a FODMAP content of maximum 0.2 gram fructans per 50 g of bread, typically maximum 0.1 gram fructans per 50 g of bread. The wholemeal can consist of wheat wholemeal.

The wholemeal bread can have a minimum fibre level of about 3 gram fibre per 50 gram bread

The invention is summarised in the following statements:

1. Use of *Kluyveromyces marxianus* for reducing FODMAP (Fermentable oligo-, di- , monosaccharides and polyols) content in wholemeal bread or wholemeal dough by 80 % compared to being prepared by a S. cerevisiae reference strain.

4.

2. The use according to statement 1, wherein *K. marxianus* is the strain with deposit number CBS6014.

3. A wholemeal yeast fermented bread characterized in a FODMAP content of maximum 0.2 or 0.1 gram fructans per 50 g of dry bread.

4. The wholemeal yeast fermented bread according to statement 3, with a FODMAP content of maximum 0.1 gram fructans per 50 g of bread on dry matter base.

5. The wholemeal yeast bread according to statement 3 or 4, in which the wholemeal consists of wheat wholemeal, or comprises at least 70, 80 or 90 % (w/w) of wheat wholemeal.

6. The wholemeal yeast bread according to any of statements 3 or 4, in which the wholemeal comprises at most 50 % (w/w) rye wholemeal.

7. The wholemeal yeast bread according to any of statements 3 to 6, with a minimum fibre level of about 3 gram fibre per 50 gram bread on dry matter base.

## DETAILED DESCRIPTION OF THE INVENTION

### LEGENDS TO FIGURES

The present invention will become more fully understood from the detailed description and the accompanying drawing which are given by way of illustration only, and thus are not limitative of the present invention.

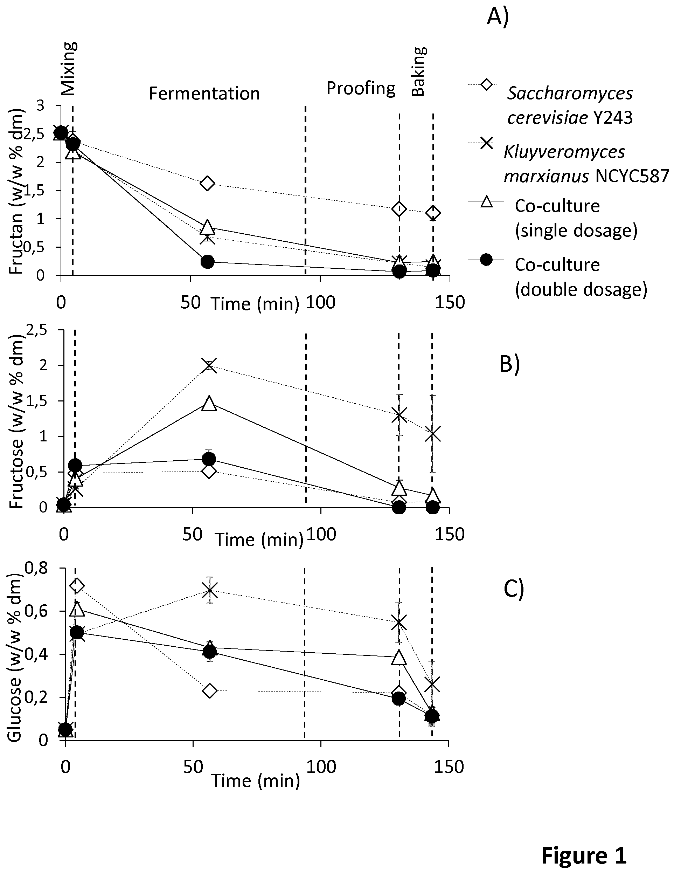

**Figure 1.** Fructan (A), fructose (B) and glucose (C) concentrations in Terroir wholemeal dough and bread samples fermented with the *S. cerevisiae* bakery strain Y243, *K. marxianus* strain NCYC587, a single dosage co-culture or a double dosage co-culture. The single dosage co-culture contains 1.06% yeast dry matter (on wholemeal basis), the double dosage contains 2.12% yeast dry matter (on wholemeal basis). Fructan/fructose/glucose concentrations are expressed as weight percentages on wholemeal dry matter base (w/w % dm). The first time point (t = 0) shows fructan/fructose/glucose concentrations in Terroir wholemeal. The last time point represents fructan/fructose/glucose concentrations in bread after baking. Vertical dashed lines separate the four consecutive steps of bread making:

mixing, fermentation, proofing and baking. Error bars are standard deviations on measurements of biological triplicates.

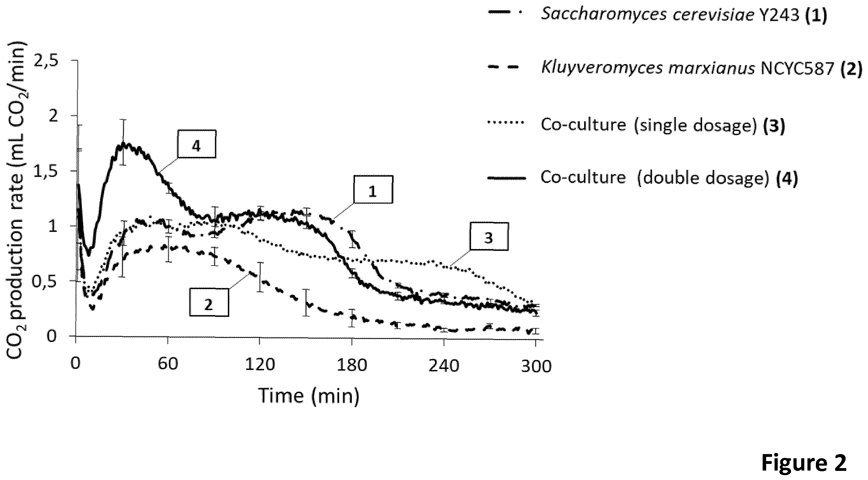

**Figure 2.** COz production rate (mL COz/min) of the *S. cerevisiae* bakery strain Y243, *K. marxianus* strain NCYC587, a single dosage co-culture and a double dosage co-culture during Terroir wholemeal dough fermentation (30°C). The single dosage co-culture contains 1.06% yeast dry matter (on wholemeal basis), the double dosage contains 2.12% yeast dry matter (on wholemeal basis). Vertical bars represent standard deviations on measurements of biological triplicates.

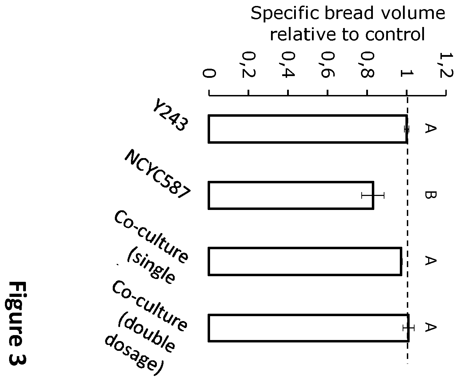

**Figure 3.** Specific bread volumes (relative to a control Terroir wholemeal bread prepared with *S. cerevisiae* strain Y243) of Terroir wholemeal breads prepared with *K. marxianus* strain NCYC587, a single dosage co-culture and a double dosage co-culture. The single dosage co-culture contains 1.06% yeast dry matter (on wholemeal basis), the double dosage contains 2.12% yeast dry matter (on wholemeal basis). Vertical bars represent standard deviations on measurements of biological triplicates. Means with different letters are significantly different (P<0.05).

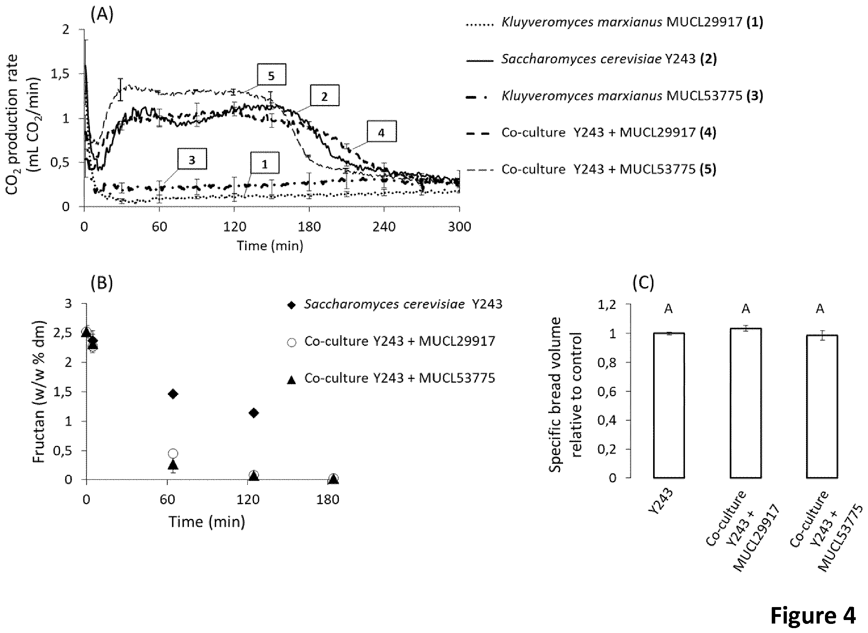

**Figure 4.** (A) COz production rate (mL COz/min) of the *S. cerevisiae* bakery strain Y243, *K. marxianus* strains MUCL29917 and MUCL53775 (1.06% yeast dry matter on wholemeal basis) and co-cultures of Y243 + MUCL29917 (2 x 1.06% yeast dry matter) and Y243 + MUCL53775 (2 x 1.06% yeast dry matter) during Terroir wholemeal dough fermentation (30°C).

(B) Fructan concentrations in dough samples fermented with *S. cerevisiae* bakery strain Y243, co-culture Y243 + MUCL29917 and co-culture Y243 + MUCL53775. Fructan concentrations were measured after 60, 120 and 180 min of fermentation and are expressed as weight percentages on wholemeal dry matter base (w/w % dm). The first time point (t = 0) shows fructan concentrations in Terroir wholemeal. The vertical dashed line indicates the time point after mixing. (C) Specific bread volumes (relative to a control Terroir wholemeal bread prepared with *S. cerevisiae* bakery strain Y243) of Terroir wholemeal breads prepared with co-culture Y243 + MUCL29917 and co-culture Y2431 + MUCL53775. Vertical bars represent standard deviations on measurements of biological triplicates. Means with different letters are significantly different (P <0.05).

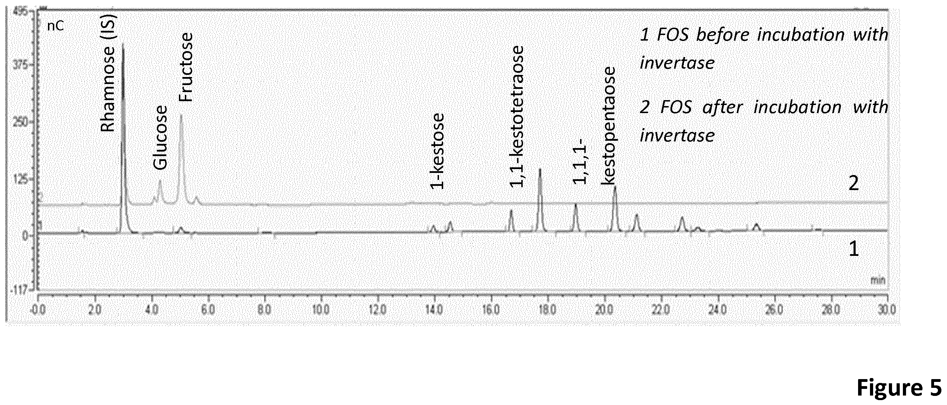

**Figure 5:** High Performance Anion Exchange Chromatography with Pulsed Amperopetric Detection (HPAEC-PAD) profile of a fructose-oligosaccharide solution [(FOS, Orafti P95, Beneo, (black line)] incubated with 100 U (on kestose, 40°C) of invertase [I4504, Sigma Aldrich (grey line)] for 2 h (50°C, pH 5). The peaks were identified after comparison with a standard solution containing rhamnose (internal standard), glucose, fructose, 1-kestose, 1,1-kestotetraose and 1,1,1-kestopentaose. The peaks in between the identified GFn-type fructans (1-kestose, 1,1-kestotetraose and 1,1,1-kestopentaose) are Fn type fructans.

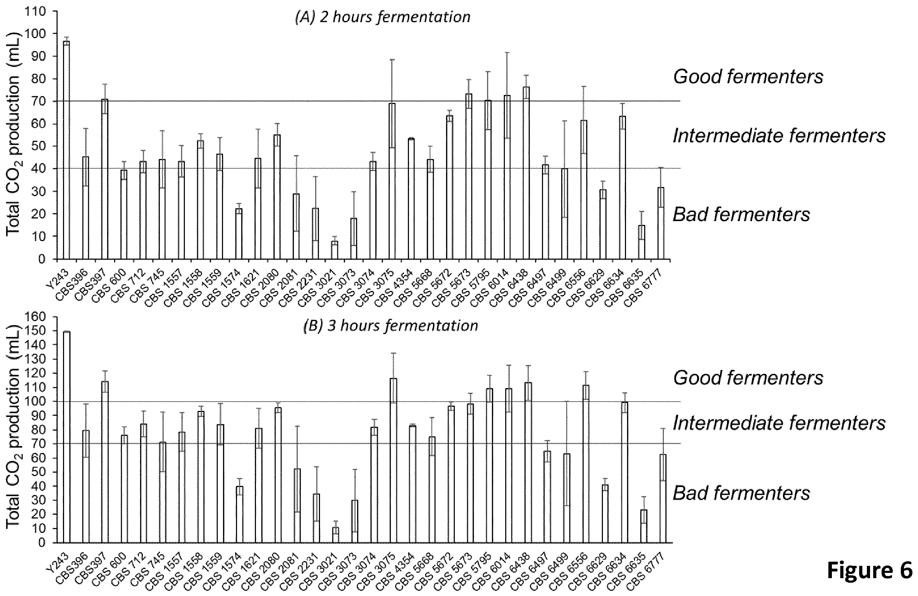

**Figure 6.** Total amount of COz (mL) produced in dough (10g scale) by the S. *cerevisiae* bakery strain (Y243) and 30 different *K. marxianus* strains (CBS collection) after (A) two hours of fermentation and (B) 3 hours of fermentation. The total amount of COz produced was measured using Risograph analysis. The strains are divided in good, intermediate and bad fermenters according to their total amount of COz produced during fermentation. Vertical bars represent standard deviations on measurements of biological duplicated.

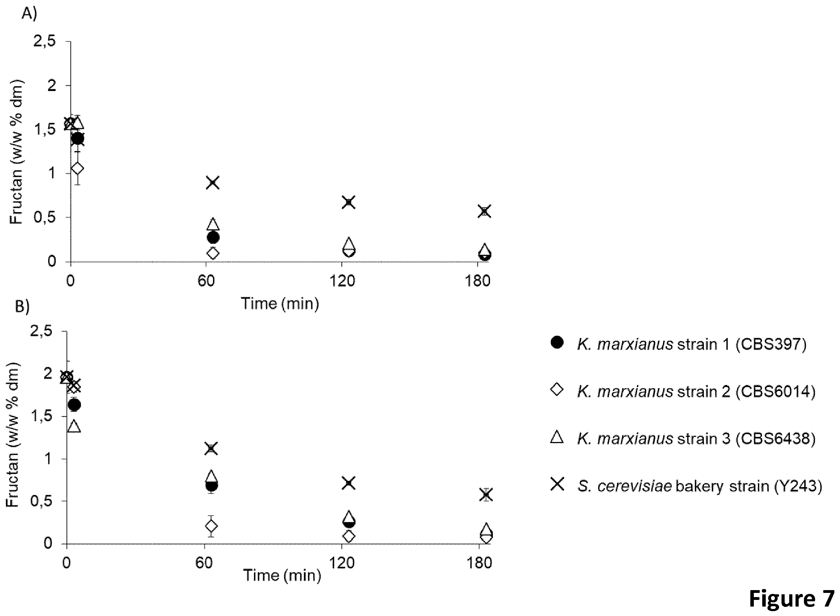

**Figure 7.** Evolution of fructan content in dough prepared with (A) Atomic flour and (B) Atomic wholemeal and fermented with *K. marxianus* strains (strain 1 to 3) and the *S. cerevisiae* bakery strain (Y243). The first time point (t = 0) was defined as the moment when mixing was started and represents the fructan concentration in (A) Atomic flour or (B) wholemeal. Fructan concentrations are expressed as weight percentages on wholemeal dry matter base (w/w % dm). Vertical bars represent standard deviations of biological triplicate measurements.

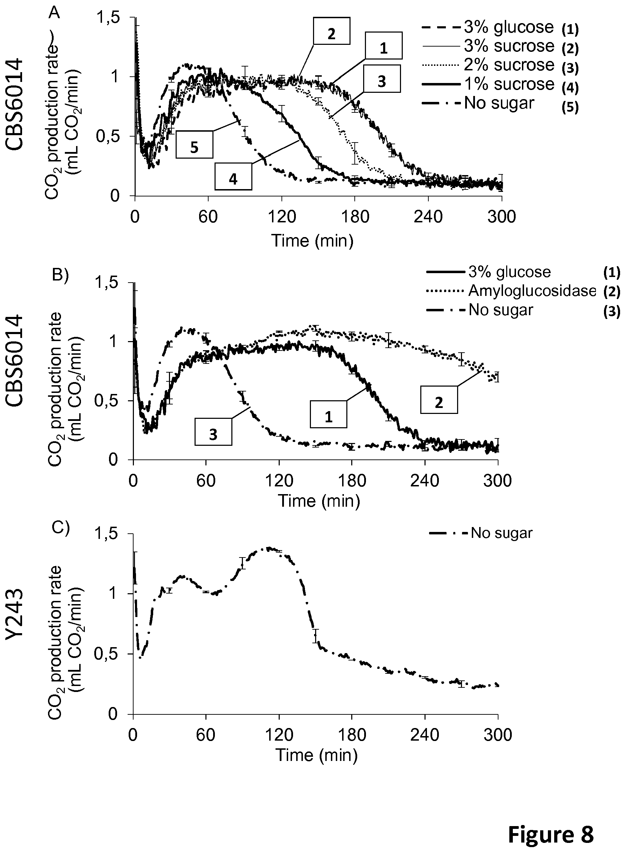

**Figure 8.** (A) COz production rate (mL COz/min) of *K. marxianus* CBS6014 in Atomic wholemeal dough with different levels of added sugars.

(B) COz production rate (mL $CO_2$/min) of *K. marxianus* CBS6014 in Atomic wholemeal dough with addition of 15U g/dm amyloglucosidase (AMG).. The COz production profile of CBS6014 in Atomic wholemeal dough with no added sugar and addition of 3% glucose is included as a control.

(C) COz production rate (mL $CO_2$/min) of *S. cerevisiae* Y243 in Atomic wholemeal dough without addition of sugars. Vertical bars represent standard deviations on biological triplicate measurements.

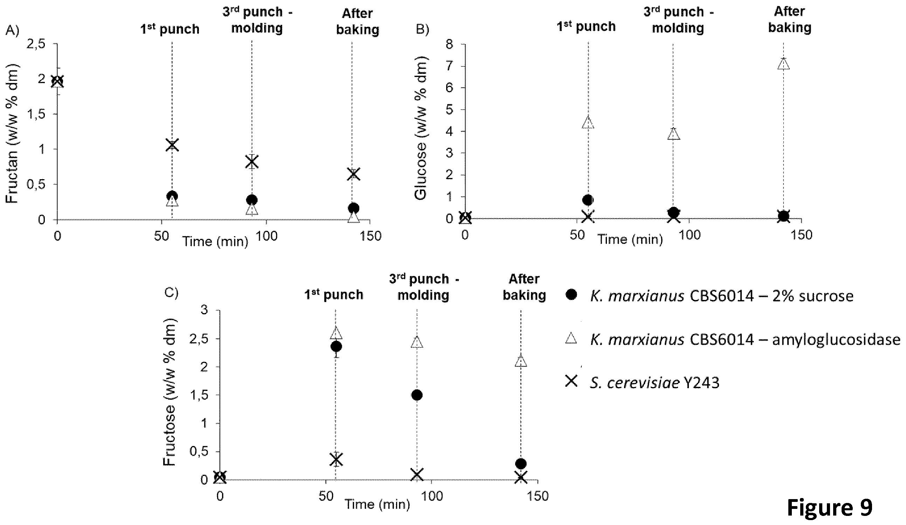

**Figure 9.** Evolution of (A) fructan, (B) glucose and (C) fructose levels in Atomic wholemeal dough during bread making. Rounds represent Atomic wholemeal dough fermented with CBS6014 and addition of 2% sucrose. Triangles represent Atomic wholemeal dough fermented with CBS 6014 and addition of 15 U/g dry matter amyloglucosidase. Crosses represent Atomic wholemeal dough fermented with *S. cerevisiae* Y243 without supplementations. The vertical dashed lines indicate the different steps during bread making (first punch, 3th punch -molding and after baking). Fructan concentrations are expressed as weight percentages on wholemeal dry matter base (w/w % dm). Vertical bars represent standard deviations on biological triplicate measurements.

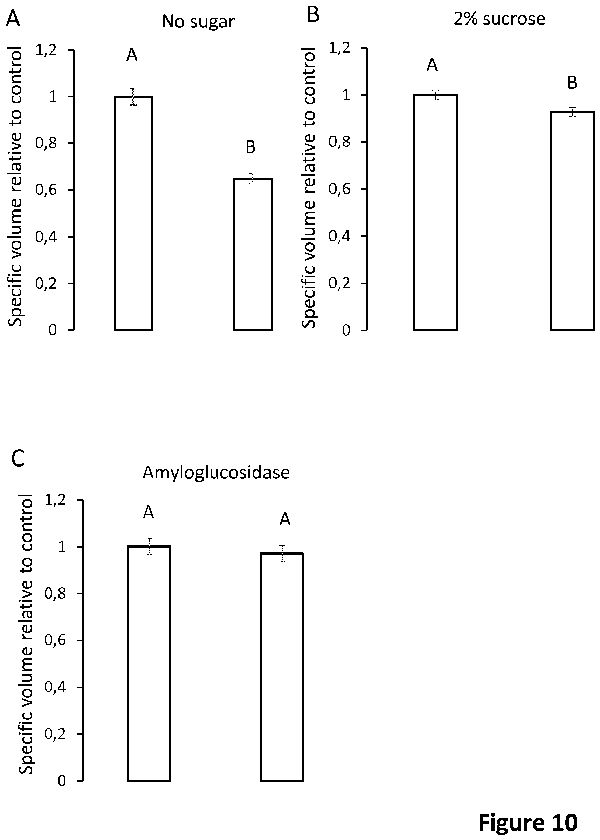

**Figure 10.** Loaf volume of breads prepared with *Kluyveromyces marxianus* CBS6014 and *Saccharomyces cerevisiae* Y243 without added sugar (A) or with addition of 2 g/100 g flour sucrose (B) or with 15 U/g dry matter amyloglucosidase (C). Loaf volume of breads prepared with CBS6014 is expressed relatively to that of bread prepared with Y243 under the same conditions. Vertical bars represent standard deviations on biological triplicate measurements. Means with the different letters are significantly different ($p<0.05$).

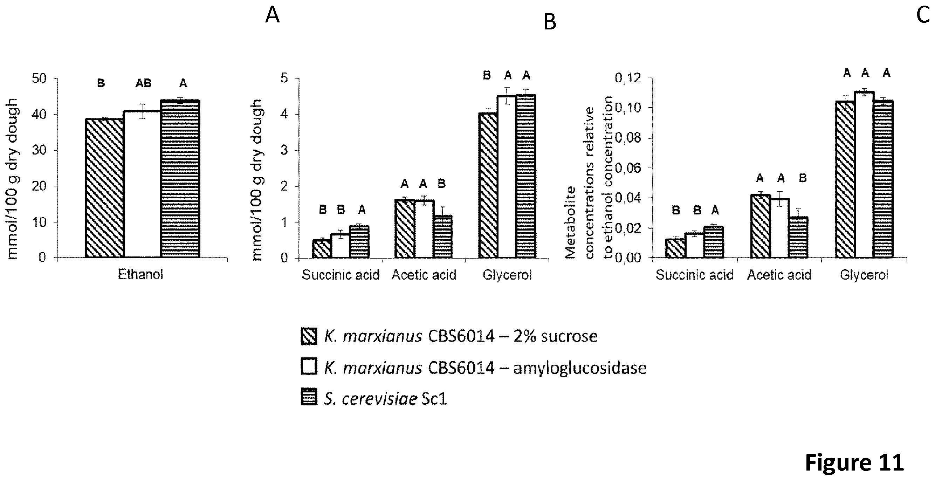

**Figure 11.** Quantification of (A) ethanol (mmol/100g dough) and (B) succinic acid, acetic acid and glycerol levels (mmol/100g dough) in Atomic wholemeal dough after fermentation (126 min) with *K. marxianus* CBS6014 and *S. cerevisiae* Y243. Sucrose (2%) or amyloglucosidase (15 U/g dry matter) were included in the dough when CBS6014 was used as starter culture (C) Succinic acid, acetic acid and glycerol levels in Atomic wholemeal dough expressed relatively to the respective ethanol concentrations. Vertical bars represent standard deviations on biological duplicate measurements. Means with the different letters are significantly different ($p<0.05$).

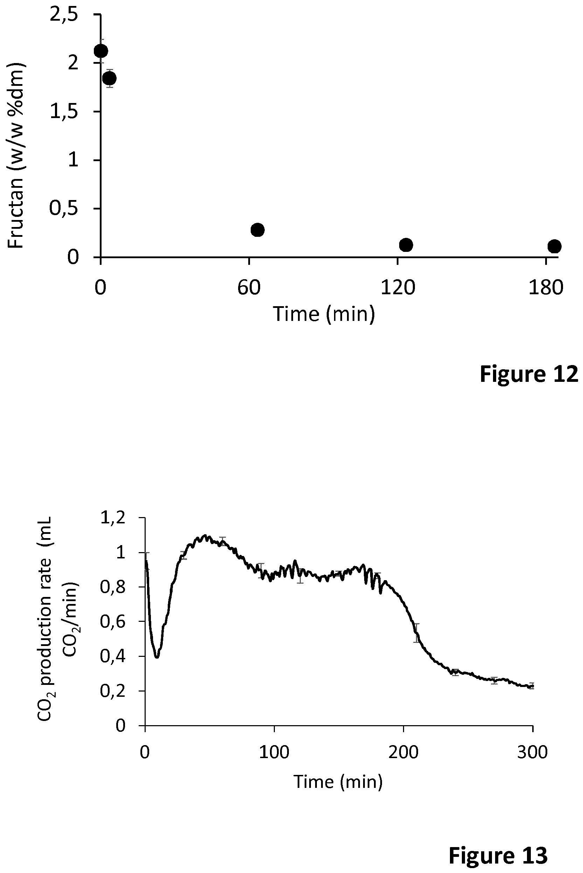

**Figure 12:** Fructan concentrations in Atomic whole meal dough samples fermented with a co-culture of *S. cerevisiae* Y243 and *K. marxianus* CBS6014. The co-culture contains a total of 1.06% yeast dry matter (on whole meal basis). Fructan concentrations are expressed as weight percentages on whole meal dry matter base (w/w % dm). The first time point (t = 0) shows fructan concentrations in Atomic whole meal. The next time points show fructan concentrations after 60, 120 and 180 min of fermentation. Vertical bars are standard deviations on measurements of biological triplicates.

**Figure 13:** COz production rate (mL COz/min) of a co-culture of *S. cerevisiae* Y243 and *K. marxianus* CBS6014 in Atomic whole meal dough. The co-culture contains a total of 1.06% yeast dry matter (on whole meal basis). Vertical bars represent standard deviations on biological triplicate measurements.

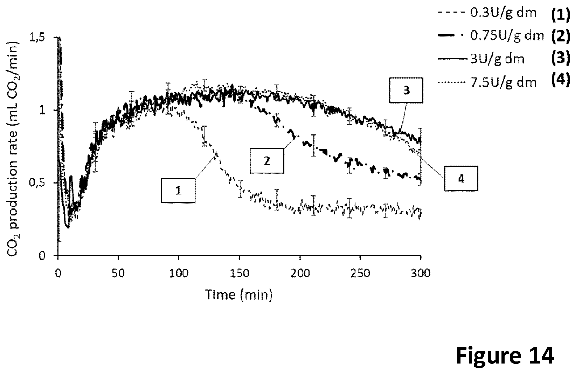

**Figure 14:** COz production rate (mL COz/min) of *K. marxianus* CBS6014 in Atomic whole meal dough with different of added dosages (0.3, 0.75, 3 and 7.5U/g dm) of amyloglucosidase (AMG). Vertical bars represent standard deviations on biological triplicate measurements.

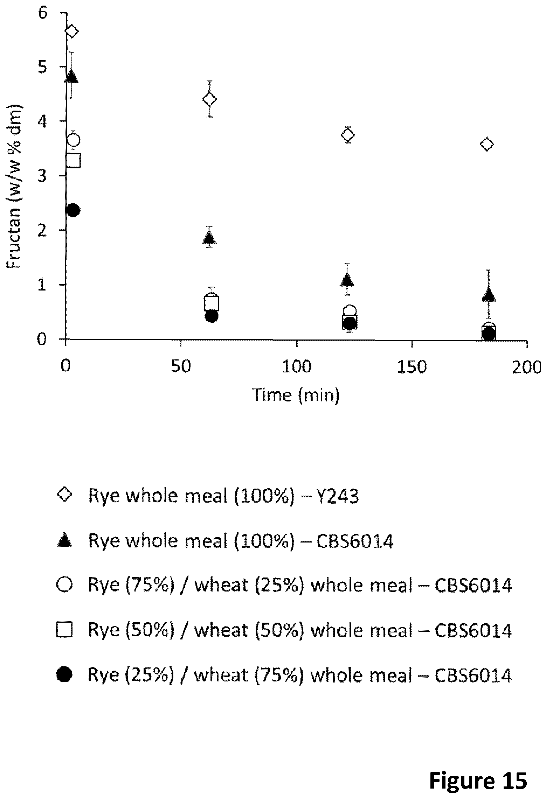

**Figure 15:** Fructan concentrations in dough samples prepared with (a mix of) rye and wheat whole meal in different ratios (100%, 75%/25%, 50%/50%, 25%/75%), fermented with *S. cerevisiae* Y243 and *K. marxianus* CBS6014. Vertical bars represent standard deviations on biological triplicate measurements.

## DETAILED DESCRIPTION OF EMBODIMENTS OF THE INVENTION

DEFINITIONS

**"Wholemeal flour"** as used in this application shall refer to flour that is partly or entirely milled from whole grains or almost-whole grains (with removal of outher layers of the grain but including part of the bran layer, endosperm and germ) from grains such as wheat or rye or oat or barley. In wholemeal flour, the individual components of the grain are ground and separated by sieving and sifting but then reconstituted to reform the wholegrain (wholemeal) flour. Optionally the separated bran fraction is further reduced in particle size before reconstitution.

**"Fibre"** as used in this application shall refer to dietary fibre and non-starch polysaccharides such as arabinoxylans, cellulose, and many other plant components such as resistant starch, resistant dextrins, inulin, lignin, chitins, pectins, beta-glucans, and oligosaccharides.

**"FODMAP"** as used in this application shall refer to "fermentable oligo-, di-, and monosaccharides and polyols". FODMAPs represent a group of short-chain carbohydrates and sugar alcohols comprising fructose, lactose, fructo- and galacto-oligosaccharides (fructans and galactans) and polyols. In this application, fructan is used as a quantitative measure for FODMAP content. Fructose is only considered as a FODMAP when fructose levels exceed glucose levels. Fructans can be measured by High performance anion exchange chromatography with pulsed amperometric detection (HPAEC-PAD). Typical concentrations of fructan in wheat wholemeal range from 0.7% on dry matter basis to 2.9% on dry matter basis. fructan content ranges between wholemeal of different plants. High fructan content is encountered in e.g. rye, with concentrations ranging between 3.6% on dry matter basis and 6.6% on dry matter basis. Fructan levels in spelt, durum wheat and triticale are similar to those found in wheat grains. Barley ($\leq$ 1.0%) and oat grains (<0.5%), on the contrary, contain lower fructan levels than wheat grains. Maize and rice do not accumulate fructans. Maize, rice and oat grains are gluten-free cereals. The invention is typically performed with wholemeal from one grain, but can be optionally be a mixture one or more grains, in different ratio's. *Kluyveromyces marxianus* strain with deposit number CBS6014 has been described in US20060257529 and is commercially available from CBS-KNAW Collections (Westerdijk Fungal Biodiversity Institute, Utrecht, Nederland). *Kluyveromyces marxianus* strain with deposit number NCYC587 has been described by McKay (1990) Lett Appl. Microbiol. 11, 41-44 and is commercially available from National Collection of Yeast Cultures (Quadram Institute Bioscience, Norwich, UK).

**"Wholemeal bread"** as used in this application shall refer to bread that is made using wholemeal flour. Typically, the methods as disclosed herein are performed with wholemeal that has not been processed containing the bran, the germ, and the endosperm in the naturally occurring proportions. The methods are equally applicable on a meal that contains up to 10 % 20 %, 30% or up to 50 % flour that has been added to wholemeal flour. Alternatively, and in contrast with the above embodiment the methods as disclosed herein can be performed on a wholemeal that has been has been sieved and wherein flour has been partially removed (e.g. up to 5, up to 10 or up to 20 % of the weight of the wholemeal has been removed). A wholemeal bread or wholemeal dough of the present invention is a product with a fructan content of at most 0.4 g, 0.3g, 0.2 g, 0.15 g or 0.1 g/ 100 g wholemeal dry matter. A wholemeal bread can be made from a dough of consisting wholemeal, yeast, water and optionally salt. Variants of a dough for a wholemeal bread may contain in addition one of more of fat (triglyceride) such as granulated fat or shortening or oil or even lard, vegetables (such as dried tomatoes, olives) or fruit (such as apples, raisins, candied fruit or dried nuts). The dough may comprise other conventional dough ingredients e.g. proteins such as milk powder, gluten and soy; eggs (either whole eggs, egg yolks or egg whites); an improver e.g. ascorbic acid, potassium bromate, potassium iodate, azodicarbonamide or ammonium persulfate; an amino-acid such as L-cysteine; a sugar; a salt such as sodium chloride, calcium acetate, sodium sulfate or calcium sulfate. The dough may further comprise an emulsifier such as mono-or diglycerides, diacetyl tartaric acid esters of mono- or diglycerides, sugar esters of fatty acids, polyglycol esters of fatty acids, lactic acid esters of monoglycerides, acetic acid esters of monoglycerides, polyoxyethylene stearates or lysolecithin. The dough as disclosed herein may be fresh, frozen or par-baked.

Bread made by the use of sourdough comprising or consisting *lactobacillus* or other bacteria such as is excluded from the scope of the present invention.

A bread prepared by sourdough has a different taste and different smell that bread that is prepared by using only yeasts for fermentation.

To distinguish over bread wherein the dry premix or the fermenting dough comprises dough, the bread of the present invention is referred to as yeast fermented bread.

"reduced" in the context of "reduced FODMAP content" shall refer to a comparison of a bread or dough prepared by a reference *Saccharomyces cerevisiae* bakery strain and a bread or a dough prepared by a *K. marxianus* strain under the same conditions, and wherein the FODMAP content in the product of the invention contains 20% less, 30 % less, 40 % less, 50 %, 60% less, 70% less, 80% less FODMAP compared to the prior art reference product. The percentage reduction is calculated as follows.

- $B_{S.cerevisiae}$ = bread prepared with *S. cerevisiae* containing the same ingredients as the bread prepared with *K. marxianus.*
- $B_{K.marxianus}$ = bread prepared with *K. marxianus* containing the same ingredients as the bread prepared with *S. cerevisiae.*
- $F_{S.cerevisiae}$ = fructan content of $B_{S.cerevisiae}$ determined using the HPAEC-PAD method.
- $F_{K.marxianus}$ = fructan content of $B_{K.marxianus}$ determined using the HPAEC-PAD method.

$$\text{Percentage reduction} = (F_{S.cerevisiae} - F_{K.marxianus})/F_{S.cerevisiae} * 100$$

"about" in the context of the present invention refers to a possible deviation of 10 % of a numeric value. For example, "about 20" is between 18 and 22, "about 60" is between 54 and 66, etc. In this context "about equal amount of two components A and B" ranges from 45 % A and 55 % B, to 55 % A and 45 % B.

The present invention relates to a wholemeal bread characterized in that the wholemeal bread has a FODMAP content of maximum 0.2 grams fructans per 50 gram bread on dry matter basis. A specific embodiment of the present invention relates to a wholemeal bread characterized in that the wholemeal bread has a FODMAP content of maximum 0.1 grams fructans per 50 gram bread on dry matter basis. In another embodiment of the present invention, the wholemeal bread with a FODMAP content of maximum 0.2 gram fructans per 50 gram bread on dry matter basis is prepared using wheat wholemeal. In another embodiment of the present invention, the wholemeal bread with a FODMAP content of maximum 0.1 gram fructans per 50 gram bread on dry matter basis is prepared using wheat wholemeal. The wholemeal bread with reduced FODMAP content can be consumed by persons suffering from IBS but also Inflammatory Bowel Disease (IBD) or non-celiac gluten sensitivity (NCGS).

As a generally accepted rule, bread which contains less the 0,2 g FODMAPS per 50 gram can be consumed by IBS patients without induction of symptoms of the disease. However, the sensitivity of IBS patients for FODMAPs is highly variable and this threshold value is only a guiding value. The lower the FODMAP levels in bread, the higher the chance that symptom induction is avoided.

**EXAMPLES**

**EXAMPLE 1** - **Materials and Methods**

*Materials.* Wheat variety Terroir was obtained from the experimental site of the Université de Liege (Agro-bio Tech, Gembloux, Belgium). Terroir wheat was milled into flour with a Buhler MLU-202 laboratory mill, with a milling yield of 69.3%. The bran and shorts fractions were further reduced in size (<500 $\mu$m) with a Cyclotec 1093 sample mill (FOSS, Höganäs, Sweden), after which they were added to the flour fraction in their original proportions to produce wholemeal. Terroir wholemeal was finally enriched with 5% vital wheat gluten with a protein content of 82.4% (w/w) (Tereos Syral, Aalst).

A fructanase mixture (E-FRMXLQ) containing exo-inulinase (2000 U/mL on kestose at 40°C) and endo-inulinase (100 U/mL on fructan at 40°C) was obtained from Megazyme (Bray, Ireland). Invertase (I4504) from *S. cerevisiae* ($\geq$300 U/mg solid on sucrose at 55°C) was obtained from Sigma-Aldrich (Bornem, Belgium). A commercial *S. cerevisiae* bakery strain (Y243) and three different *K. marxianus* strains (NCYC587, MUCL29917 and MUCL53775MUCL53775) were obtained from the collection of the VIB Laboratory for Systems Biology (KU Leuven, Belgium). These *K. marxianus* strains are known to secrete inulinase.

Yeast extract and balanced peptone were provided by Lab M (Brussels, Belgium). All other chemicals and reagents were purchased from Sigma-Aldrich and were of analytical grade.

*Wholemeal characterization.* The protein content (N x 5.7) of Terroir wholemeal was determined using an automated Dumas protein analysis system (EAS, VarioMax N/CN, Elt, Gouda, The Netherlands) following an adapted version of AOAC method 990.03. Terroir wholemeal had a protein content of 11.4% on dry matter (dm) base. The damaged starch content of Terroir wholemeal (7.89 $\pm$ 0.23% dm) was determined using a colorimetric assay (Megazyme) based on AACCI method 76-31. The falling number of Terroir wholemeal (297 $\pm$ 2 s) was determined according to AACCI method 56-81.03. Optimal baking absorption and mixing time were determined using Farinograph (Brabender, Duisburg, Germany) and Mixograph (National Manufacturing, Lincoln, NE, US) analyses according to AACCI Methods 54-21.02 and 54-40.02, respectively. All measurements were carried out in triplicate.

*Preparation and growth of yeast cells.* Yeast precultures, made by suspending a yeast colony in 5 mL YPD (1.0% w/v yeast extract, 2.0% w/v balanced peptone and 2.0% w/v glucose), were shaken (250 rpm) overnight at 30°C. After 16 h, 3 mL of the preculture was used to inoculate 300 mL YPD in a baffled Erlenmeyer flask. This second culture was shaken (250 rpm) overnight at 30°C. The next morning, the optical density (OD) at 595 nm was measured with a microplate reader (Bio-Rad laboratories, Nazareth, Belgium). For dough preparation, the yeast cells were harvested at an OD of 1.1-1.2 (*S. cerevisiae*) and 1.2-1.3 (*K. marxianus*) by centrifugation (3 min, 870g) using a benchtop centrifuge (model EBA 21, Hettich Lab Technology, Massachusetts, USA). Preliminary experiments showed that harvesting the cells at these OD values resulted in maximal fermentation rates in dough. Cells were washed with sterile dH2O before inoculation in dough. Experiments were always performed with three biological replicates. The growth profiles of the *S. cerevisiae* bakery strain and the K. *marxianus* strains were determined with the Bioscreen C (Thermo Fisher Scientific, Aalst, Belgium) during 96 h of incubation at 30 °C with continuous shaking. The OD was measured every 15 min.

*Dough and bread making.* Dough was prepared in triplicate using the straight dough method, using the following formula: 10.0 g wholemeal (on a 14% moisture basis), 1.5% (w/w) sodium chloride, and 5.3% (w/w) freshly harvested yeast, unless specified otherwise. The moisture content of the freshly harvested yeast pellets was approx. 80-85% (compared with 65-70% for commercial fresh block yeast). The ingredients were mixed in a 10 g pin bowl mixer (National Manufacturing) for 4 min 30 s. Fermentation and proofing were performed in a fermentation cabinet (National Manufacturing) at 30°C and 90% relative humidity for 90 min and 36 min, respectively. Fermenting doughs were punched at 52, 77 and 90 min of fermentation. Dough samples were taken after mixing (4.5 min), after the first punch (56.5 min) and after proofing (130.5 min). The dough samples were frozen in liquid nitrogen, lyophilized and ground with a laboratory mill to a powder prior to saccharide analysis. After proofing, doughs were baked for 13 min at 232°C in a rotary oven (National Manufacturing). Breads were subsequently cooled for 2 h and their volume was determined with a Volscan profiler (Stable Micro Systems, Godalming, Surrey, UK). After the volume measurement, breads were frozen in liquid nitrogen, lyophilized and ground with a laboratory mill to a powder prior to saccharide analysis.

*Gas production measurement.* The volume of gas produced in dough as a function of fermentation time was measured using a Risograph instrument (National Manufacturing). Doughs were prepared as described earlier and were left to ferment for 300 min at 30°C in the Risograph chambers. Gas production was measured every minute.

*Quantification of mono-, di- and trisaccharides.* Mono-, di-, and trisaccharides were extracted with hot water and subsequently quantified by high-performance anion-exchange chromatography with pulsed amperometric detection (HPAEC-PAD). Separation of saccharides by HPAEC-PAD is based on the weakly acidic nature of carbohydrates, giving highly selective separations at high pH using a strong anion-exchange stationary phase. HPAEC-PAD can directly quantify carbohydrates without derivatisation and with simple sample treatment. Saccharide concentrations were expressed as weight percentages on wholemeal dry matter base (% dm). The saccharides present in Terroir wholemeal were previously quantified and described. Glucose, fructose, and maltose concentrations in Terroir wholemeal were ≤0.1% dm for every saccharide. Sucrose and raffinose concentrations in Terroir wholemeal were 0.94 ± 0.04% dm and 0.29 ± 0.04% dm, respectively.

*Quantification of fructan.* Quantification of fructan in the lyophilized dough and bread powders was performed. A mild acid treatment is used for fructan hydrolysis, followed by analysis of the released glucose and fructose with high performance anion exchange chromatography with pulsed amperometric detection (HPAEC-PAD). Glucose and fructose concentrations before and after fructan hydrolysis were compared and used for quantification of total fructan level. Saccharide concentrations were expressed as weight percentages on wholemeal dry matter base (% dm). The fructan content in Terroir wholemeal was 2.52 ± 0.11% dm.

A bread after baking and cooling typically contains about 40 % of water. Thus a weight of a bread of 100 g is during drying reduced to 60 gram dry matter. This conversion allows to compare dry matter based weight as used in the present invention with weight as expressed in prior art publications.

*Incubation of different substrates with fructan-degrading enzymes.* Three different substrates (fructo-oligosaccharides (FOS), inulin and wheat grain fructans) were incubated with invertase (I4504) and inulinase (E-FRMXLQ) to assess the specificity of these enzymes towards substrates with a different DP. Fructo-oligosaccharides (FOS, Orafti P95, Beneo) had a DP range of 2 to 8. Inulin HP (Orafti HP, Beneo) had an average DP ≥23. Enzymes and substrates were dissolved in sodium acetate buffer (50 mM, pH 5). Wheat grain fructans (average DP of 5.8 ± 0.2) were extracted from wholemeal (variety Atomic). An aliquot of 150 μL of FOS or inulin solution (0.5 mg/mL) was incubated with 100 U of enzyme solution [invertase (70 μL) or inulinase (50 μL)] for 2 h at 50°C. After incubation, the samples were boiled for 10 min to inactive the enzymes. Wheat grain fructans were extracted from wholemeal by addition of 15 mL boiling water and subsequently 1 h of shaking in a water bath at 80°C. After extraction, samples were centrifuged and an aliquot of 150 μL of the supernatant was incubated with 100 U of enzyme solution [invertase (70 μL) or inulinase (50 μL)] for 2 h at 50°C. After incubation, the samples were boiled for 10 min to inactive the enzymes. Fructan levels were determined as described above and experiments were always performed in triplicate.

*Statistical analysis.* Dough or bread samples were prepared in triplicate and saccharide concentrations were quantified once in each dough or bread sample, unless specified otherwise. The data were analysed using statistical software JMP Pro 12. Significant differences were determined by one-way analysis of variance using JMP Pro software 12 (SAS Institute, Cary, NC, US), with comparison of mean values using the Tukey test ($\alpha$=0.05).

**EXAMPLE 2** - **Analysis of FODMAP levels in dough and bread samples fermented with different yeast cultures.**

Fructan degradation was evaluated during bread making The different yeast cultures that were used as leavening agents, represented in Table 1, include S. *cerevisiae* and *K. marxianus* monocultures, but also co-cultures of both species.

**Table 1:** The composition of the different yeast cultures that were used

| Yeast culture | Yeast Strain code | Composition | Yeast dosage (g yeast pellet/100 g wholemeal flour) | Yeast dosage (g yeast dry matter/ 100 g wholemeal flour) |
|---|---|---|---|---|
| Monoculture Y243 | Y243 | *S. cerevisiae Y243* | 5.30% | 1.06% |
| Monoculture NCYC587 | NCYC587 | *K. marxianus NCYC587* | 5.30% | 1.06% |
| Co-culture (single dosage) | Y243 | *S. cerevisiae Y243* | 2.65% | 0.53% |
| | NCYC587 | *K. marxianus NCYC587* | 2.65% | 0.53% |
| Co-culture (double dosage) | Y243 | *S. cerevisiae Y243* | 5.30% | 1.06% |
| | NCYC587 | *K. marxianus NCYC587* | 5.30% | 1.06% |

Figure 1A shows fructan levels in dough and bread samples measured after mixing (4.5 min), after the first punch (56.5 min), after proofing (130.5 min) and after baking (143.5 min). S. *cerevisiae* bakery strain Y243 degraded the fructan present in wholemeal slower and to a lower extent than *K. marxianus* strain NCYC587 and the co-cultures.

After mixing, only minor differences were detected in fructan levels between doughs fermented with the different yeast cultures. After the first punch, however, fructan levels present in the different dough samples were ranked as follows: Y243 (1.6% dm) > single dosage co-culture (0.8% dm) > NCYC587 (0.7% dm) > double dosage co-culture (0.2% dm). Dough samples prepared with the double dosage co-culture contained a higher amount of yeast, which means that more fructan degrading enzymes (invertase and inulinase) were produced, explaining the fast degradation of fructan in these dough samples.

After proofing, the fructan level in dough fermented with *S. cerevisiae* Y243 was still above 1% dm (1.2% dm). In dough fermented with the NCYC587 mono-culture, the single dosage co-culture and the double dosage co-culture, fructan levels were respectively 0.2% dm, 0.2% dm and <0.1% dm. Fructan levels were not significantly reduced during the baking phase of bread making. This means that breads prepared with Y243 contained >1% dm fructan, while fructan levels in breads prepared with NCYC587, the single dosage co-culture and the double dosage co-culture were 0.2% dm, 0.2% dm and <0.1% dm, respectively. Foods containing >0.2 g of fructans in an average serving quantity of the food are considered at-risk for inducing symptoms in IBS subjects. Based on the proposed threshold value (0.2 g per serve, and one serve is 50 g of bread), fructan levels in breads prepared with NCYC587 and the co-cultures were low enough to minimize symptom induction in FODMAP sensitive people.

More than 90% of the fructans initially present in wholemeal were degraded by NCYC587 and the co-cultures, while only 56% was degraded when a mono-culture of Y243 was used. Inulinase thus seems much more effective in degrading wheat grain fructans than invertase during bread making, which might be explained by the different substrate specificity of both enzymes.

Next to fructan, also fructose levels in doughs and breads prepared with the different cultures were analysed. Fructose levels in dough and bread samples prepared with the different cultures, measured at different time points during the bread making process, are represented in Figure 1B. Relatively high fructose levels were present in all dough samples after the first punching step. Fructose concentrations were the highest in dough fermented with the NCYC587 monoculture, which might indicate that this culture fermented more slowly compared with the other cultures. Indeed, it might be possible that NCYC587 consumed the hydrolysis products of fructan more slowly than the other cultures. Fructose concentrations were the lowest in doughs fermented with the Y243 mono-culture and the double dosage co-culture. The latter might be explained by the high fermentation rate of this culture and hence the fast consumption of sugars, as a relatively high dosage of yeast was present in this co-culture. The low fructose concentration in dough fermented with the Y243, on the other hand, might be related to the lower fructan degrading capacity of this strain. In all cases, except for breads prepared with the NCYC587 mono-culture, fructose levels were $\leq$0.2% dm.

Figure 1C represents glucose levels in dough and bread samples prepared with the different cultures at different time points during the bread making process. Glucose is not a FODMAP, but its presence in food products can enhance the absorption of fructose. Glucose levels in doughs fermented with NCYC587 and the co-cultures (Figures 1B and 1C) were always lower than fructose levels, indicating that fructose was present in excess. This observations might be explained by the fact that (i) less glucose is released during fermentation compared with fructose as fructan mainly consists of fructose units and/or (ii) glucose is the preferred sugar source of yeast over fructose. After baking, however, both glucose and fructose levels in the breads were very low ($\leq$0.3% dm) except for breads prepared with the NCYC587 monoculture. Breads prepared with NCYC587 contained fructose levels ($\pm$1% dm) that were clearly higher than glucose levels ($\pm$0.3% dm).

**EXAMPLE 3** - **Comparison of CO$_2$ production rate of different yeast cultures during dough fermentation.**

In order to prepare qualitative breads with the different yeast cultures, their COz production rate in dough should be sufficiently high. Therefore, the COz production rate (mL CO$_2$/min) of the *S. cerevisiae* bakery strain Y243, *K. marxianus* strain NCYC587 and co-cultures of both strains was analyzed during Terroir wholemeal dough fermentation (Figure 2).

The COz production rate of *S. cerevisiae* strain Y243 reached 1.0 mL/min after 30 min of fermentation. After approx. 70 min of fermentation, a slight drop in COz production rate was observed. After the drop, the CO$_2$ production rate returned back to its maximal rate and only dropped to lower levels after approx. 180 min of fermentation due to depletion of maltose.

The COz production rate of *K. marxianus* strain NCYC587 in Terroir wholemeal dough was lower than that of *S. cerevisiae* strain Y243. After approx. 30 min of fermentation, a maximal COz production rate of 0.7-0.8 mL/min was reached. This lower COz production rate is correlated with the lower rate of fructose and glucose consumption by NCYC587 compared with Y243, as shown in Figures 1B and 1C.

The COz production rate in dough fermented with NCYC587 already dropped to levels <0.5 mL/min after 120 min of fermentation.

As the COz production rate of NCYC587 was relatively low and dropped quickly, co-cultures containing both Y243 and NCYC587 seem advisable for the production of breads with a sufficiently high loaf volume. The COz production rate (mL CO$_2$/min) of the co-cultures in Terroir wholemeal dough is represented in Figure 2. The COz production rate of the single dosage co-culture in Terroir wholemeal dough was similar to the COz production rate of the Y243 mono-culture during the first 2 h of fermentation. After 2 h of fermentation, the COz production rate of the single dosage co-culture dropped slightly to 0.7-0.8 mL/min, while the COz production rate of the Y243 mono-culture remained high (1.1 mL/min). Despite the small drop in COz production after 2 h of fermentation, the single dosage co-culture seems more suitable for bread making than the *K. marxianus* monoculture, as the productive fermentation time of the first was longer and because it reached higher COz production rates.

The COz production rate of the double dosage co-culture was very high ($\approx$1.7 mL/min) during the first hour of fermentation. As the double dosage co-culture contains more (fermenting) yeast cells, it is assumed that fructose and glucose, the primary fermentation substrates, will be depleted earlier in doughs fermented with this co-culture, which is confirmed by the data represented in Figures 1B and 1C. The depletion of glucose and fructose was also reflected in a drop in COz production after 60 min of fermentation. Around approx. 75 min of fermentation, the COz production rate of the double dosage co-culture reached similar rates as the Y243 monoculture ($\approx$1 mL/min). This might indicate that the *S. cerevisiae* part of the culture was consuming maltose and therefore produced COz, while the *K. marxianus* part of the culture was not fermenting anymore. The COz production rate of the double dosage co-culture remained around 1 mL/min until approx. 150 min of fermentation, after which it dropped, probably due to sugar depletion.

**EXAMPLE 4** - **Bread volume of breads prepared with different yeast cultures.**

The volume of breads prepared with NCYC587 and the co-cultures were compared with the volume of bread prepared with the commercially used *S. cerevisiae* strain Y243 (Figure 3). The volume of breads prepared with Y243 and the co-cultures was not significantly different. Indeed, the production of COz by these cultures during proofing (90-126 min) was comparable and sufficient for optimal loaf volume. Breads prepared with the NCYC587 mono-culture, had a significantly lower volume than breads prepared with Y243.

**EXAMPLE 5** - **Fructan degrading capacity of different *K. marxianus* strains.**

*K. marxianus* strain NCYC587 degraded the wheat grain fructans present in Terroir wholemeal almost completely (>90%) during fermentation. To see if this also is the case for other strains, two other *K. marxianus* strains were tested. First, the COz production rate of the two strains (MUCL29917 and MUCL53775) was tested in Terroir wholemeal dough (Figure 4A). The strain codes of MUCL29917 and MUCL53775 were KV3266 and KV3278, respectively. Since the COz production rate of both strains was very low ($\leq$0.3 mL/min), co-cultures of 5.3% *K. marxianus* (MUCL29917 or MUCL53775) and 5.3% *S. cerevisiae* Y243 were used for dough and bread making trials. The moisture content of the yeast pellets was approx. 80%, indicating that the amount of yeast dry matter added to dough was 2.1% on wholemeal basis. The COz production rate of the co-cultures was comparable or slightly higher than that of Y243, indicating that the production of COz in the co-culture was mainly attributed to the presence of the *S. cerevisiae* cells. Co-cultures of Y243 with MUCL29917 or MUCL53775 degraded all the fructans present in Terroir wholemeal dough after 2 h of fermentation (Figure 4B). This indicates that the combination of invertase and inulinase synthesized by these co-cultures was sufficient to degrade the wheat grain fructans present in Terroir wholemeal. Bread volumes of breads prepared with co-cultures of Y243 with MUCL29917 or MUCL53775 were not significantly different from the volume of breads prepared with *S. cerevisiae* Y243 alone (Figure 4C). Fructose and fructan levels present in breads prepared with the co-cultures were $\leq$0.1% dm.

The results indicate that co-cultures of *S. cerevisiae* and two other *K. marxianus* strains were able to fully degrade wheat grain fructans during bread making. The *K. marxianus* strains showed low COz production rates in dough when they were used as mono-culture. Therefore, addition of *S. cerevisiae* cells is advised for sufficient COz production and optimal loaf volumes. This means that the *K. marxianus* part of the co-cultures in this case mainly contributed to fructan degradation and not to dough leavening. Addition of (high dosages of) enzymes (inulinase) might give the same results.

A wide variety of other *K. marxianus* strains were tested for their (i) CO2 production rate and (ii) fructan degrading capacity (see Figure 6 and Figure 7). It was shown that K. *marxianus* CBS6014 was able to (i) produce high amounts of COz during dough fermentation and (i) degrade all the fructans present in wheat wholemeal. Co-cultures of *S. cerevisiae* Y243 and *K. marxianus* CBS6014 (2 x 0.53g yeast dry matter/100g wholemeal flour) had a sufficiently high fermentation rate (up to 1 mL/min for >150 min, Figure 13) during dough fermentation. The fructan content in wholemeal flour dough fermented with this co-culture was <0.2% dm after two hours of fermentation (Figure 12).

**EXAMPLE 6** - **Differences between *K. marxianus* inulinase and S. *cerevisiae* invertase.**

*K. marxianus* inulinase degraded the fructans present in wholemeal faster and to a greater extent than *S. cerevisiae* invertase. The enzyme has an octameric structure, best described as a tetramer of dimers. The dimeric structure sets steric constraints that limit the access to the active site of oligosaccharides of more than four units. Since the mean DP of wheat grain fructans is 4 to 5 and the maximal DP was reported to be 19, it is possible that a part of the fructans present in wheat do not fit properly in the active site of invertase. To verify this hypothesis, different substrates (inulin - mean DP $\geq$ 23, FOS - DP 2-8 and wheat grain fructans) were incubated for 2 h with an overdose of pure invertase at 50°C and pH 5, the optimal conditions for invertase (Table 2). As a control, the different substrates were also incubated with a fructanase mixture, containing endo- and exo-inulinase, that is able to hydrolyse higher DP fructans such as inulin completely.

**Table 2:** The degradation of different substrates (fructo-oligosaccharides (FOS), inulin, wheat grain fructans) with invertase and inulinase..

| Substrate | Before incubation | After incubation with invertase | Degradation by invertase | After incubation with inulinase | Degradation by inulinase |
|---|---|---|---|---|---|
| FOS (DP 2 - 8) (mg/mL) | 0.54 $\pm$ 0.01 | 0.00 $\pm$ 0.00 | 100 % | 0.00 $\pm$ 0.00 | 100 % |
| Inulin (DP $\geq$ 23) (mg/mL) | 0.52 $\pm$ 0.01 | 0.30 $\pm$ 0.02 | 42 % | 0.00 $\pm$ 0.01 | 100 % |
| Wheat grain fructans (mg/mL) | 0.51 $\pm$ 0.04 | 0.04 $\pm$ 0.02 | 92 % | 0.02 $\pm$ 0.03 | 96 % |

FOS, inulin and wheat grain fructan levels were measured before and after incubation with invertase and inulinase (2 h, pH 5, 50°C), and the total degradation of substrate (%) was calculated. Experiments were performed in triplicate and standard deviations are shown in the table

Quite surprisingly, the results indicated that invertase was able to fully degrade FOS with a DP of 2 to 8. HPAEC-PAD profiles of the FOS solution before and after incubation revealed that also the FOS with a DP>4 were completely degraded by invertase (Figure 5). When wheat grain fructans from wholemeal were incubated with pure invertase, more than 90% of the fructans were degraded, which is a lot more than what was observed during dough fermentation. The part that was not degraded (8%) might have a DP that is too high for hydrolysis by invertase. As expected, invertase degraded inulin only partially (42% degradation), which can be explained by its lower specificity towards higher DP fructans. Inulinase degraded inulin and FOS completely, and also 96% of the wheat grain fructans were degraded by inulinase (Table 2). These results confirm that inulinase has a higher specificity towards fructans with a high DP like high DP inulin, as reported. Yet, also invertase can degrade oligosaccharides with a DP>4.

Additionally, the results also showed that pure invertase degraded more than 90% of extracted wheat grain fructans in a buffered solution, revealing that invertase is also able to degrade branched fructan structures, as wheat grains contain branched graminan- and neo-type fructans. These observations might indicate that also other factors than only substrate specificity limit the degradation of wheat grain fructans by *S. cerevisiae* during bread dough fermentation.

Another reason for the lower activity of *S. cerevisiae* invertase towards wheat grain fructans during dough fermentation might be that invertase is mainly retained in the cell wall, while inulinase has both a cell wall associated and a secreted form. It might be possible that fructans with a high DP cannot penetrate the cell wall, and are therefore not accessible for invertase. When an overdose of commercial *S. cerevisiae* block yeast (16% on wholemeal basis) was added, however, 93% of the fructans present in Terroir wholemeal were degraded during dough fermentation (2 h), indicating that at least 93% of the wheat grain fructans were able to penetrate the yeast cell wall. Addition of very high dosages of yeast ($\geq$ 3 x 5.3%) results in almost complete fructan degradation. These high dosages would, however, result in excessive COz production during proofing and collapse of the dough structure, and might lead to off-flavours.

**EXAMPLE 7** - **Materials and Methods**

*Materials.* Wheat variety Atomic was obtained from the experimental site of the Université de Liege (Agro-bio Tech, Gembloux, Belgium). Atomic wheat was milled into flour and wholemeal with a Buhler MLU-202 laboratory mill. The milling yield was 69%. To produce wholemeal, the bran and shorts fractions were further reduced in size (<500 $\mu$m) with a Cyclotec 1093 sample mill (FOSS, Höganäs, Sweden), after which they were added to the flour fraction in their original proportions. Gluten with a protein content of 82.4 % (w/w) were obtained from Tereos Syral (Aalst, Belgium).

A commercial *S. cerevisiae* bakery strain (Y243) was obtained from the collection of the VIB Laboratory for Systems Biology (KU Leuven, Belgium). A total of 30 different *K. marxianus* strain were obtained from the Laboratory of Molecular Cell Biology (KU Leuven, Belgium). Yeast extract and balanced peptone were provided by Lab M (Brussels, Belgium). All other chemicals and reagents were purchased from Sigma-Aldrich and were of analytical grade.

*Flour and Wholemeal characterization.* The protein content (N x 5.7) of Atomic flour and wholemeal was determined using an automated Dumas protein analysis system (EAS, VarioMax N/CN, Elt, Gouda, The Netherlands) following an adaptation of the AOAC method 990.03. Atomic flour and wholemeal had a protein content of 10.6% and 13.1% on dry matter (dm) base, respectively. The damaged starch level of Atomic flour and wholemeal was determined using a colorimetric assay provided by Megazyme, based on AACCI method 76-31. The Falling number (FN) of Atomic flour and wholemeal was determined according to AACCI method 56-81.03. Optimal baking absorption and mixing time were determined using Farinograph (Brabender, Duisburg, Germany) and Mixograph (National Manufacturing, Lincoln, NE, USA) analyses according to AACCI Methods 54-21.02 and 54-40.02, respectively. All measurements were carried out in triplicate.

*Preparation and growth of yeast cells.* Yeast precultures, made by suspending a yeast colony in 3 mL YPSuc (1.0% w/v yeast extract, 2.0% w/v balanced peptone and 2.0% w/v sucrose), were shaken (250 rpm) for 8 h at 30°C. After 8 h, 1.5 mL of the preculture was used to inoculate 200 mL YPSuc in an Erlenmeyer flask. This second culture was shaken (250 rpm) overnight at 30°C. After 16 h, the optical density (OD) at 595 nm was measured with a microplate reader (Bio-Rad laboratories, Nazareth, Belgium). For dough preparation, the yeast cells were harvested at an OD of 1.1 (*S. cerevisiae* strain) or 1.2-1.3 (*K, marxianus* strains) by centrifugation (3 min, 870g) using a benchtop centrifuge (model EBA 21, Hettich Lab Technology, Massachusetts, USA). These OD values were previously determined to give the highest fermentation rates in dough. Cells were washed with sterile dH2O before inoculation in dough. Experiments were always performed with three biological replicates.

The growth profile of *S. cerevisiae* bakery strain and the *K. marxianus* strains was determined with the Bioscreen C (Thermo Fisher Scientific, Aalst, Belgium) during 96 hours of incubation at 30 °C with continuous shaking. The OD was measured every 15 min.

*Dough and bread making.* Dough was prepared in triplicate according to a variation of the straight dough method using the following formula: 10.0 g flour or wholemeal (on a 14% moisture basis), 1.5% (w/w) sodium chloride, and 5.3% (w/w) freshly harvested yeast, unless specified otherwise. Depending on the type of experiment, sugars or enzymes were included in the bread making recipe. The ingredients were mixed in a 10 g pin bowl mixer (National Manufacturing) for 3 min 10 s.

Fermentation and proofing were performed in a fermentation cabinet (National Manufacturing) at 30°C and 90% relative humidity for 90 min and 36 min, respectively. Fermenting doughs were punched at 52, 77 and 90 min of fermentation. Dough samples were taken at several time points during fermentation and frozen in liquid nitrogen, lyophilized and ground with a laboratory mill to a powder prior to saccharide analysis. After proofing, doughs were baked for 13 min at 232°C in a rotary oven (National Manufacturing). Breads were subsequently cooled for 2 h and their volume was determined with a Volscan profiler (Stable Micro Systems, Godalming, Surrey, UK). After volume measurement, breads were frozen in liquid nitrogen, lyophilized and ground with a laboratory mill to a powder prior to saccharide analysis. Breads were always prepared in triplicate, with three biological replicates of each strain.

*Gas production measurement.* The volume of gas produced in dough during fermentation was measured using a Risograph instrument (National Manufacturing). Doughs were prepared as described in paragraph earlier and were left to ferment for 300 min at 30°C in the Risograph chambers. Gas production was measured every minute. Doughs were always prepared in triplicate, with three biological replicates of each strain.

*Quantification of mono-, di- and trisaccharides.* Mono-, di-, and trisaccharides were extracted with hot water and subsequently quantified by high-performance anion-exchange chromatography with pulsed amperometric detection (HPAEC-PAD). Separation of saccharides by HPAEC-PAD is based on the weakly acidic nature of carbohydrates, giving highly selective separations at high pH using a strong anion-exchange stationary phase. HPAEC-PAD can directly quantify carbohydrates without derivatisation and with simple sample treatment. Saccharide concentrations were expressed as weight percentages on flour or wholemeal flour dry matter base (% dm).

*Quantification of fructan.* Quantification of fructan in the lyophilized dough and bread powders was performed. A mild acid treatment is used for fructan hydrolysis, followed by analysis of the released glucose and fructose with high performance anion exchange chromatography with pulsed amperometric detection (HPAEC-PAD). Glucose and fructose concentrations before and after fructan hydrolysis were compared and used for quantification of total fructan level. Saccharide concentrations were expressed as weight percentages on flour or wholemeal flour dry matter base (% dm).

*Quantification of fermentation metabolites.* Dough samples were extracted by blending the dough with dHzO (two times the volume of the final weight of the dough after fermentation) for 30 s with a Waring 8011E blender (Warring Products, Torrington, CT, US). The resulting batter was centrifuged with an Eppendorf centrifuge 5415D (Eppendorf AG, Hamburg, Germany) (11,000g, 3 min). The supernatant of the extracted dough samples was filtered with a Millex-HP 0.22 μm polyethersulfone membrane (Millipore, Carrigtwohill, Ireland) and stored at -20°C until further analysis.

Ethanol, glycerol, acetic acid and succinic acid in the dough extracts were quantified using ion-exclusion HPLC using an LC-20AT modular HPLC system (Shimadzu, Kyoto, Japan). Separation of the metabolites was carried out with an ion-exclusion ROA-organic acids guard (50 x 7.8 mm) and analytical (300 x 7.8 mm) column (Phenomenex, Torrance, CA, US) eluted with a $H_2SO_4$ solution (2.50 mM) at a flow rate of 0.60 mL/min. The guard and analytical column were kept at 60°C. Doughs were always prepared in triplicate, with three biological replicates of each strain.

*Sensory analysis.* Breads prepared with different yeast cultures were subjected to sensory analysis using the triangle test. First, breads were prepared on a 10 g scale using the procedure of Shogren and Finney[35] as described earlier. After cooling of the breads, slices of bread crumb (1 ± 0.05 g) that contained no crust were served in small plates at room temperature. Each plate contained a number and the serving order was randomized for each panel member. The panelists were asked to identify which of the samples was different from the others, based first only on their aroma and secondly based on their taste. Water was provided for neutralization of taste in between the testing of samples. The panelists were also asked if they had a preference for one of samples, and to explain their decision using descriptors. To detect significant differences in aroma and taste, the results of the sensory analysis were analyzed statistically using the "Critical Number of Correct Response in a Triangle Test" table. This table gives the minimum number of correct responses required for significance at the stated $\alpha$-level, for the corresponding number of respondents (n). The assumption of "no difference" is rejected if the number of correct responses is greater than or equal to the tabled value.

*Statistical analysis.* Dough and bread making experiments were always performed with three biological replicates, and saccharide concentrations were quantified once in each dough or bread sample, unless specified otherwise. Significant differences were determined by one-way analysis of variance using JMP Pro software (SAS Institute, Cary, NC, US), with comparison of mean values using the Tukey test ($\alpha$=0.05).

### EXAMPLE 8 - **Atomic flour and wholemeal characterization.**

The fructan content of Atomic wholemeal (1.96 ± 0.19% dm) was higher than the fructan content of Atomic flour (1.57 ± 0.10% dm). The same counted for sucrose, with a concentration of 0.97 ± 0.04% dm in Atomic wholemeal compared with only 0.19 ± 0.01% dm in Atomic flour. Glucose and fructose levels were very low ($\leq$ 0.05% dm) in both Atomic flour and wholemeal. The damaged starch level of Atomic flour and wholemeal was 7.39 ± 0.21% dm and 6.31 ± 0.05% dm, respectively. The Falling Number, which is correlated with $\alpha$-amylase activity and therefore maltose levels in dough, was higher for Atomic wholemeal (303 ± 7s) than for Atomic flour (255 ± 2s).

### EXAMPLE 9 - **Fermentation rate of *K. marxianus* strains in Atomic flour dough supplemented with glucose.**

In order to select good bread dough fermenting *K. marxianus* strains, the COz production rate of the 30 different *K. marxianus* strains was measured in Atomic flour dough supplemented with 3% glucose. Glucose and not sucrose was chosen as sugar source in dough because the first selection criterion for selecting the strains was their COz production rate in dough and not their capacity to degrade saccharides like sucrose. The 30 *K. marxianus* strains were divided in three classes based on their total amount of COz produced in Atomic flour dough with 3% glucose after 2 and 3 hours of fermentation: good fermenters, intermediate fermenters and bad fermenters (Figure 6). From the good fermenters, the three best fermenting and most reproducible strains were selected for analysis of their fructan degrading capacity.

### EXAMPLE 10 - **Fructan degrading capacity of selected *K. marxianus* strains in Atomic flour and wholemeal dough.**

The fructan degrading capacity of the three selected *K. marxianus* strains was evaluated in Atomic flour and Atomic wholemeal dough (Figure 7). Both in flour and wholemeal dough, 3% glucose was included in the bread making recipe since the *K. marxianus* strains cannot ferment maltose and will therefore not properly ferment without extra substrates like glucose. Glucose was not included in the bread making recipe when *S. cerevisiae* was used. The three *K. marxianus* strains were referred to as strain 1 to 3 (strain 1 = CBS 397, strain 2 = CBS 6014, strain 3 = CBS 6438).

All *K. marxianus* strains degraded the fructan present in Atomic flour and wholemeal to a greater extent than the *S. cerevisiae* bakery strain (Figure 7). Especially strain 2 degraded the fructans present in the dough very quickly, with nearly no fructan left ($\leq$ 0.2% dm) after 60 min fermentation in Atomic flour dough as well as in Atomic wholemeal dough. These observations suggest that *K. marxianus* strain 2 (CBS6014) can be used to produce FODMAP-free breads. Therefore, this strain was selected to further optimize the growth medium and the bread making recipe that is the most suitable for bread production with a *K. marxianus* strain as leavening agent.

### EXAMPLE 11 - **Optimization of bread making recipe using *K. marxianus* CBS6014 as a leavening agent.**

*K. marxianus* CBS6014 quickly degraded the fructan present in both Atomic wheat flour and wholemeal, with fructan levels <0.2% dm after 2 h fermentation (Figure 7). Addition of 3% glucose to dough ensured that enough fermentable substrates were present for sufficient production of COz during fermentation. As yeast cells have a higher preference for glucose over fructose, addition of 3% glucose hampered the consumption of fructose and therefore high fructose levels (1.46 $\pm$ 0.32% and 0.77 $\pm$ 0.03% for Atomic wholemeal and flour, respectively) were still present in the dough after 2 h of fermentation. This is undesirable when aiming at producing breads that are low in FODMAPs, as fructose is also a FODMAP and the cut-off value for inducing symptoms is around 0.5 g/100 g. To avoid high rest levels of fructose in bread, the addition of alternative sugar sources or sugar releasing enzymes was tested. These tests were only performed in Atomic wholemeal as wholemeal generally contains higher FODMAP levels than flour.

In a first step, the COz production rate of *K. marxianus* CBS6014 in Atomic wholemeal dough with different sucrose levels was evaluated and compared with the COz production rate of *S. cerevisiae* Y243 in dough (without sugar) (Figure 8A). Addition of sucrose to dough instead of glucose decreases the glucose/fructose ratio in the dough, which means that yeast strains need to start consuming fructose faster. The results revealed that addition of 2% sucrose was enough to ensure high COz production rates during the proofing period, i.e. when the doughs are in their final stage of fermentation in their baking mould. Although the COz production rate of *S. cerevisiae* strains Y243 was still higher during the second hour of fermentation (Figure 8C), differences were small. Secondly, evaluation of fructan levels at different time points during the bread making process showed that both fructan, fructose and glucose levels were $\leq$0.3% dm after baking (Figure 9A). This means that both fructan and fructose levels were under their estimated cut-off value of inducing symptoms, which is respectively of 0.2 g per serve and 0.5 g/100 g. Although it is known that the presence of sucrose results in a slower degradation of fructan by *S. cerevisiae* invertase, *K. marxianus* CBS6014 degraded the fructan present in Atomic wholemeal very quickly even when sucrose was added to dough. Indeed, 92% of the fructans present in Atomic wholemeal were degraded by CBS6014 during bread making, while this was only 67% for Y243.

The amount of fermentable sugars in the dough with 2% added sucrose was exactly high enough to ensure high fermentation rates by CBS6014 during proofing ($\pm$ 2 h fermentation), but afterwards the fermentation rate dropped. This means that the level of fermentable substrates was nearly depleted after proofing, explaining the low glucose and fructose levels in the final bread ($\leq$0.3% dm) (Figures 9B and 9C). In breads fermented with Y243 without added sugar, glucose and fructose levels were also very low ($\leq$0.2% dm) as *S. cerevisiae* yeast cells consume glucose and fructose first before they switch to maltose consumption.

In a next step, the supplementation of Atomic wholemeal with amyloglucosidase (AMG) instead of glucose was evaluated. Amyloglucosidase releases high amounts of glucose from damaged starch derived from dextrines during mixing and fermentation but also during baking. As the inactivation temperature of yeast ($\pm$ 55°C) is lower than that of AMG (65°C), glucose will be released during baking without subsequent consumption by the yeast cells. The presence of glucose in the final bread might make sure that the presence of fructose does not induce symptoms in patients suffering from IBS. Indeed, when present with glucose, fructose is absorbed more efficiently across the small intestinal epithelium. This response is believed to be related to the insertion of a specific transporter, GLUT-2, into the apical membrane of the enterocytes. Figure 8B shows the COz production rate of *K. marxianus* strain CBS 6014 in Atomic wholemeal dough with supplementation of AMG. The addition of AMG resulted in higher COz production rates than the presence of 3% glucose, indicating that both dosages of AMG released relatively high amounts of glucose (>3%) that serve as fermentable substrates for CBS6014. As with addition of 2% sucrose, the COz production rate of CBS6014 in dough with addition of AMG was lower than that of Y243 in a control dough, but differences were small (Figure 8C). Evaluation of fructan levels at different time points during the bread making process revealed that fructan levels in dough supplemented with AMG were already very low during fermentation and $\leq$0.1% dm after baking (Figure 9A). This means that up to 97% of the fructans initially present in Atomic wholemeal were degraded during bread making. Glucose levels in the bread with AMG supplementation were relatively high ($\pm$ 7%) (Figure 9B), and it is suggested that glucose is released during baking as glucose levels were higher after baking than after the 3th punch. As glucose is released continuously by AMG, fructose levels in the dough remained high ($\pm$ 2%) (Figure 9C). This indicates that the fructose released by inulinase from fructan and sucrose was only consumed to a very limited extent by the yeast cells. This confirms that *K. marxianus* CBS6014 has a greater preference for glucose over fructose, which is also described for *S. cerevisiae.*

Because the release of glucose during fermentation is correlated with the amount of amyloglucosidase added, it was analyzed whether the addition of lower dosages of amyloglucosidase (< 15U/g dm) can result in a sufficiently high COz production rate but lower final bread glucose levels. Results showed that a dosage of 0.75U/g dm amyloglucosidase was sufficient for COz production rates >1 mL/min for approx. 150 min (Figure 14). It is assumed that this lower dosage results in equal loaf volumes but will have less effect on the taste (i.e. sweetness) compared with the dosage of 15U/g dm.

To resume, it can be concluded that addition of sucrose or amyloglucosidase offers strategies to produce FODMAP-free breads with a mono-culture of *K. marxianus* CBS6014 as leavening agent. The amount of sucrose should be sufficient to ensure optimal COz production rates during proofing, but not higher as fructose will remain in the bread when too much sucrose is added. Amyloglucosidase can be added to dough to ensure high COz production rates during proofing and high bread glucose levels.

**EXAMPLE 12** - **Loaf volume of breads prepared with *Kluyveromyces marxianus* CBS6014 as a leavening agent.**

Wheat variety Atomic, selected for its high fructan content, had a low protein content, which explains why breads prepared with Atomic flour and wholemeal had relatively low loaf volumes. Indeed, breads prepared with Y243 and CBS6014 had comparable but low loaf volumes, making it difficult to draw conclusion about the potential of CBS6014 for bread making. Therefore, we included 5% gluten with a protein content of 82.4% w/w dm in the bread making recipe. Breads were prepared with Atomic wholemeal with either no sugar, 2% sucrose or amyloglucosidase (the same dosage as used previously). As expected, the loaf volume of breads prepared with *K. marxianus* CBS6014 was lower than that of breads prepared with the control *S. cerevisiae* strain when no sugar or amyloglucosidase was added (Figure 10). As the COz production rate of CBS6014 already dropped after 60 min when no sugar was added (Figure 8A), no leavening occurred during proofing (90-126 min) leading to very low loaf volumes. When sucrose or amyloglucosidase was added, the COz production rate of CBS6014 was still high during proofing (Figures 8A and 8B). In the case of 2% sucrose, the loaf volume of breads prepared with CBS6014 was still significantly lower than that of breads prepared with Y243, but the difference was small (only 7% compared with 35% when no sugar was added) (Figure 10). The lower volume of breads prepared with CBS6014 and 2% sucrose compared with that of breads prepared with Y243 might be explained by the slightly lower fermentation rate of CBS6014 (Figure 8). When AMG was included, no differences in bread volume were observed when CBS6014 and Y243 were compared (Figure 10).

**EXAMPLE 13** - **Evaluation of secondary metabolites produced by K. *marxianus* CBS6014 in dough.**

The production of secondary metabolites by yeast can affect dough rheology by altering the dough pH. Dough acidification causes an increase in net protein charge, which can lead to increased protein solubility and facilitated protein unfolding. This can results in dough that shows an increase in resistance to extension, but decreased extensibility and dough stability. Therefore, the metabolites produced by *K. marxianus* CBS6014 were measured and compared with the metabolites produced by *S. cerevisiae* Y243. Metabolites (ethanol, glycerol, succinic acid and acetic acid) were measured in Atomic wholemeal dough after 126 min fermentation. Sucrose (2%) or AMG was added to dough when the CBS6014 strain was used, while no sugar was added when the *S. cerevisiae* bakery strain was used, as this strain does not need extra sugars to ferment at high rates. Results showed that metabolite production by CBS6014 in dough was comparable with metabolite production by *S. cerevisiae* Y243 (Figure 11). Ethanol levels after 2 h of fermentation were slightly higher in doughs fermented with the *S. cerevisiae* strain (Figures 9A and 9B). When expressed relatively to the respective ethanol concentration in the samples, no differences in glycerol production were observed between CBS6014 and Y243, which might indicate that both suffer to the same extent from the osmotic stress conditions present in the dough (Figure 11C). Succinic acid levels were lower compared with acetic acid levels, both in doughs fermented with CBS6014 as in doughs fermented with Y243 (Figure 11B). The level of acetic acid was higher in doughs fermented with CBS6014 compared with Y243, while the opposite was observed for succinic. These effects were also observed when acid concentrations were expressed relatively to the respective ethanol concentration in the dough sample (Figure 11C).

As the production of acids during fermentation is correlated with the dough Ph, the pH of doughs fermented with Y243 (no sugar) was compared with the pH of doughs fermented with CBS6014 (2% sucrose). After 2 h of fermentation, the pH of dough fermented with the CBS6014 (5.55 $\pm$ 0.01) was slightly higher compared with that of dough fermented Y243 (5.44 $\pm$ 0.01).

**EXAMPLE 14** - **Evaluation of bread aroma when using *K. marxianus* CBS6014 as a leavening agent.**

Yeast cultures and breads were subjected to sensory analysis using the triangle test. The goal of this test was to evaluate if the aroma as well as the taste of the bread prepared using CBS 6014 could be discriminated by a consumer test panel from that produced by the conventional bakery strain (Y243).

First, the aroma of harvested Y243 and CBS6014 yeast cells was evaluated by a test panel of 35 persons using the triangle test. Panellists were asked to identify which one of the three harvested yeast culture was different from the others, based on their aroma (smell). The CBS 6014 yeast culture was perceived as having a different aroma by 25 out of 35 panellists. This means that the aroma of yeast culture CBS6014 was significantly different from that of yeast culture Y243 (a = 0.001). The panellists described the aroma of CBS6014 as stronger, more fruity and sour apple. The aroma of Y243 was described as less pronounced, less strong, softer, less aroma.

In a next step, the aroma and taste of bread made using CBS6014 and Y24 was compared. For bread making, Atomic wholemeal was used. Sucrose (2%) was added when CBS6014 was used as a leavening agent for to gain satisfactory CO2 production. No sugar was added when Y243 as this strain is able to consume maltose. The total glucose and fructose content in breads prepared with CBS6014 and Y243 was < 0.3% (Figures 8C and 8D), which means that the sucrose added was consumed and will not affect the bread taste.

The results of these sensory tests demonstrated that the taste of bread prepared with CBS6014 was significantly different from that of bread prepared with Y243 (a = 0.10). A total of 12 out of 23 could recognize differences in the taste of bread prepared with CBS6014 compared with that of bread prepared with Y243. When asked to describe the difference in taste, 8 out of 12 panellists said that the differences in taste were difficult to describe and very small.

When asked to their preference, one panellist had a preference for bread prepared with Y243, while three had a preference for bread prepared with CBS6014. The other 19 panellists declared to have no preference. No one declared that the taste of one of the bread samples was unacceptable for consumption. For the aroma of the breads, 11 out of 23 panellists recognized a difference between breads prepared with CBS6014 and Y243 (a = 0.20). As with the taste, difference were described to be very small and hard to define.

**EXAMPLE 15** - **Fructan levels in wheat and rye wholemeal dough mixtures fermented with S. cerevisiae Y243 and *K. marxianus* CBS6014**

The only cereal source that has a higher fructan content than wheat is rye, in which fructan levels of more than 6% were observed. Therefore, *K. marxianus* CBS6014 was inoculated into dough prepared from rye wholemeal and rye/wheat wholemeal mixtures to evaluate fructan degradation. It was shown that *K. marxianus* CBS6014 could not completely degrade the fructans present in a 100% rye meal dough. After 2 hours of fermentation, still more than 1% dm fructan was present (Figure 15). When dough was prepared from mixtures of rye wholemeal and wheat wholemeal (75% rye, 50% rye or 25% rye), fructan levels were respectively 0.53% dm, 0.33% dm and 0.31% dm after 2 hours of fermentation.

### Claims

1. Use of *Kluyveromyces marxianus* yeast for reducing FODMAP (Fermentable oligo-, di-, monosaccharides and polyols) content of wholemeal bread or wholemeal dough by 80 % compared to being prepared by a *S. cerevisiae reference* strain.

2. The use according to claim 1, wherein *K. marxianus* is the strain with deposit number CBS6014.

3. A wholemeal *Kluyveromyces* marxianus yeast fermented bread, **characterized in** a fructan content of maximum 0.2 gram fructans per 50 g of bread on dry matter base.

4. The wholemeal yeast fermented bread according to claim 3, with a fructan content of maximum 0.1 gram fructans per 50 g of bread on dry matter base.

5. The wholemeal yeast fermented bread according to claim 3 or 4, in which the wholemeal consists of wheat wholemeal, or comprises at least 70, 80 or 90 % (w/w) of wheat wholemeal.

6. The wholemeal yeast fermented bread according to any one of claims 3 to 5, in which the wholemeal comprises at most 50 % (w/w) rye wholemeal.

7. The wholemeal yeast fermented bread according to any of claims 4 to 6, with a minimum fibre level of 3 gram fibre per 50 gram bread on dry matter base.

### Patentansprüche

1. Verwendung von *Kluyveromyces marxianus*-Hefe zum Reduzieren eines Gehalts von FODMAP (fermentierbare Oligo-, Di-, Monosaccharide und Polyole) von Vollkornbrot oder Vollkornteig um 80 % im Vergleich zu einer Herstellung mit einem *S. cerevisiae-Referenz*stamm.

2. Verwendung nach Anspruch 1, wobei *K. marxianus* der Stamm mit der Ablagerungszahl CBS6014 ist.

3. Ein mit *Kluyveromyces* marxianus-Hefe fermentiertes Vollkornbrot, **gekennzeichnet durch** einen Fruktangehalt von maximal 0,2 Gramm Fruktanen pro 50 g Brot auf Trockenmassebasis.

4. Mit Hefe fermentiertes Vollkornbrot nach Anspruch 3, mit einem Fruktangehalt von maximal 0,1 Gramm Fruktanen pro 50 g Brot auf Trockenmassebasis.

5. Mit Hefe fermentiertes Vollkornbrot nach Anspruch 3 oder 4, wobei das Vollkorn aus Weizenvollkorn besteht oder mindestens 70, 80 oder 90 Gew.-% Weizenvollkorn umfasst.

6. Mit Hefe fermentiertes Vollkornbrot nach einem der Ansprüche 3 bis 5, wobei das Vollkorn höchstens 50 Gew.-% Roggenvollkorn umfasst.

7. Mit Hefe fermentiertes Vollkornbrot nach einem der Ansprüche 4 bis 6, mit einem Mindestfasergehalt von 3 Gramm Faser pro 50 Gramm Brot auf Trockenmassebasis.


**Revendications**

1. Utilisation d'une levure *Kluyveromyces marxianus* permettant de réduire de 80 % une teneur en FODMAP (oligo-, di-, monosaccharides et polyols fermentescibles) d'un pain de farine complète ou d'une pâte de farine complète par comparaison avec une préparation à base d'une souche de *référence S. cerevisiae.*

2. Utilisation selon la revendication 1, dans laquelle *K. marxianus* est la souche portant le numéro de dépôt CBS6014.

3. Pain de farine complète fermenté à la levure *Kluyveromyces marxianus,* **caractérisé par** une teneur en fructosanes de maximum 0,2 gramme de fructosanes pour 50 g de pain sur une base de matière sèche.

4. Pain de farine complète fermenté à la levure selon la revendication 3, avec une teneur en fructosanes de maximum 0,1 gramme de fructosanes pour 50 g de pain sur une base de matière sèche.

5. Pain de farine complète fermenté à la levure selon la revendication 3 ou 4, dans lequel la farine complète est constituée de farine complète de blé, ou comprend au moins 70, 80 ou 90 % (P/P) de farine complète de blé.

6. Pain de farine complète fermenté à la levure selon l'une quelconque des revendications 3 à 5, dans lequel la farine complète comprend au maximum 50 % (P/P) de farine complète de seigle.

7. Pain de farine complète fermenté à la levure selon l'une quelconque des revendications 4 à 6, avec un taux minimal de fibres de 3 grammes de fibres pour 50 grammes de pain sur une base de matière sèche.

Figure 1

Figure 2

**Figure 3**

Figure 4

**Figure 5**

1 FOS before incubation with invertase

2 FOS after incubation with invertase

(A) 2 hours fermentation

Good fermenters

Intermediate fermenters

Bad fermenters

(B) 3 hours fermentation

Good fermenters

Intermediate fermenters

Bad fermenters

**Figure 6**

EP 3 668 321 B1

Figure 7

- ● *K. marxianus* strain 1 (CBS397)
- ◇ *K. marxianus* strain 2 (CBS6014)
- △ *K. marxianus* strain 3 (CBS6438)
- ✕ *S. cerevisiae* bakery strain (Y243)

Figure 8

Figure 9

EP 3 668 321 B1

**Figure 10**

Figure 11

**Figure 12**

**Figure 13**

Figure 14

◇ Rye whole meal (100%) – Y243

▲ Rye whole meal (100%) – CBS6014

○ Rye (75%) / wheat (25%) whole meal – CBS6014

□ Rye (50%) / wheat (50%) whole meal – CBS6014

● Rye (25%) / wheat (75%) whole meal – CBS6014

**Figure 15**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015117182 A
- FR 2988565
- WO 2016113465 A
- US 20060257529 A

**Non-patent literature cited in the description**

- **ZIEGLER et al.** *J. Functional Foods,* 2016, vol. 25, 257-266
- **PLESSAS et al.** *Food Chem.,* 2006, vol. 106, 985-990
- **LAAITIKAN.** *Aliment Pharmacol Ther.,* 2016, vol. 44, 460-470
- **MCKAY.** *Lett Appl. Microbiol.,* 1990, vol. 11, 41-44